(19) Europäisches Patentamt
European Patent Office
Office européen des brevets

(11) Publication number: 0 207 735 B1

(12) # EUROPEAN PATENT SPECIFICATION

(45) Date of publication of patent specification: 21.06.89

(51) Int. Cl.[4]: C 07 D 493/04, C 09 K 15/06, C 09 K 15/08

(21) Application number: 86304954.0

(22) Date of filing: 26.06.86

(54) ## Method of manufacturing phenol-type natural antioxidants.

(30) Priority: 26.06.85 JP 140056/85

(43) Date of publication of application: 07.01.87 Bulletin 87/02

(45) Publication of the grant of the patent: 21.06.89 Bulletin 89/25

(84) Designated Contracting States: DE FR GB IT

(56) References cited:
GB-A-2 010 273

CHEMICAL ABSTRACTS, vol. 102, no. 7, February 18, 1985, Columbus, Ohio, USA. TAKEMOTO OIL AND FAT CO.,"Production of antioxidants", page 369, column 1, abstract no. 59 552j & Jpn. Kokai Tokkyo Koho JP 59157086

(73) Proprietor: Takemoto Yushi Kabushiki Kaisha
2-5, Minato-machi
Gamagouri-shi Aichi-ken (JP)

(72) Inventor: Namiki, Mitsuo
2-175 Yashirodai Meito-ku
Nagoya-shi Aichi-ken (JP)
Inventor: Osawa, Toshihiko
2-5-5-205 Chiyodabashi Chikusa-ku
Nagoya-shi Aichi-ken (JP)
Inventor: Fukuda, Yasuko
3-10-1 Ikegami-cho Chikusa-ku
Nagoya-shi Aichi-ken (JP)
Inventor: Ozaki, Tatsuhiko
6-74, Eiraku-cho
Nishio-shi, Aichi-ken (JP)

(74) Representative: Ablewhite, Alan James et al
MARKS & CLERK 57/60 Lincoln's Inn Fields
London WC2A 3LS (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 207 735 B1

Courier Press, Leamington Spa, England.

## Description

This invention relates to a method of manufacturing phenol-type natural antioxidants containing analogs of sesamin or sesamolin from processed sesame seed products.

Sesame seed oil is known to contain not only sesamolin and sesamin which are natural antioxidants peculiar to sesame seeds, but also tocopherols which are also found widely in other vegetable oils. These natural antioxidants are known to make a significant contribution to the stability of sesame seed oil against oxidation. Little is known, however, about other natural antioxidants which contribute to the stability of sesame seed oil against oxidation, and there has been hardly any attempt made to extract such materials in an industrially advantageous manner. The present invention thus relates to a method of manufacturing such natural antioxidants in an industrially advantageous manner.

The present inventors have earlier disclosed that various aglycones with antioxidative properties can be obtained by enzymatic hydrolysis of glycosides obtainable by extraction from processed sesame seed products by using β-glucosidase (Japanese Patent Tokkai 59—157173). Among the aglycones obtained are analogs of sesamin or sesamolin such as tetrahydro-1-[6-hydroxy-3,4-(methylenedioxy)-phenyl]-4-[3,4-(methylenedioxy)-phenyl]-1H,3H-furo(3,4-C] furan (a compound represented later by A), tetrahydro-1-[3-methoxy-4-hydroxyphenyl]-4-[3,4-(methylenedioxy)-phenyl]-1H,3H-furo[3,4-C] furan (a compound represented later by B), tetrahydro-1-[3-methoxy-4-hydroxyphenoxy]-4-[3,4-(methylenedioxy)-phenyl]-1H,3H-furo[3,4-C] furan (a compound represented later by D), etc.

Since the aforementioned aglycones exist within processed sesame seed products as glycosides combined with various types of saccharides, the yield of aglycones by the aforementioned conventional method is low. The method also requires complicated operations and is not appropriate for industrial applications.

It is therefore an object of this invention to provide a new method of manufacturing phenol-type natural antioxidants which eliminates the aforementioned problems of the conventional methods.

In view of the situation described above, the present inventors have investigated an industrially feasible means for obtaining natural antioxidants containing analogs of sesamin or sesamolin from sesame seeds. They have made an unexpected discovery that the concentration of such analogs of sesamin or sesamolin increases not only several times but several tens of times and moreover a large amount of the compound A which is hardly contained at all in the original sesame seed product, is produced if processed products of sesame seeds are treated with an acid catalyst. A new method of manufacturing phenol-type natural antioxidants containing analogs of sesamin or sesamolin has thus been established. Stated briefly, this invention teaches a method of manufacturing phenol-type natural antioxidants containing analogs of sesamin or sesamolin characterized in that a processed product of sesame seeds is subjected to treatment with an acid catalyst and then, the required substances are separated by physical processing means such as extraction, distillation and/or adsorption.

The processed sesame seed products which may be used according to the present invention include the following sesame oils and paste-like substances: unrefined sesame seed oils extracted mechanically, for example, by processing from raw or roasted sesame seeds; unrefined sesame seed oils extracted from such seeds by using an organic solvent such as hexane; refined sesame seed oils obtained from these unrefined sesame seed oils by chemical or physical processing such as deoxidation, bleachings and filtering; and paste-like materials obtained by crushing or milling seeds of the aforementioned types.

The acid catalysts which may be used according to this invention include the following Bronsted acids, Lewis acids and solid catalysts having the functions of an acid catalyst: various inorganic and organic Bronsted acids such as hydrochloric acid, sulfuric acid, phosphoric acid, boric acid and p-toluenesulfonic acid; Lewis acids such as aluminum chloride, titanium chloride, tin chloride and boron trifluoride, and solid catalysts having the functions of an acid catalyst such as acid clay, activated clay, zeolite, silica-titanium oxide and cation exchange resins. The above may be used singly or two or more may be used in combination. For convenience of operation, however, it is preferable to use a solid catalyst with the functions of an acid catalyst. The acid catalyst processing may be carried out either by adding an acid catalyst to a processed sesame seed product and, if necessary, heating and stirring the mixture (from room temperature to about 210°C) or by heating and stirring in a solvent system as a solution or dispersion (temperature up to the boiling point of the solvent).

Many methods are available to the present invention for carrying out the concentration and separation of the phenol-type natural antioxidants after the acid catalyst processing step. These include the following physical methods of extraction, distillation and/or adsorption which can be used together in appropriate combinations.

For extraction, a polar organic solvent (such as methanol), in which sesame seed oil (triglyceride of fatty acid) had low solubility may be used to extract the desired material after the processed sesame seed products have been treated with an acid catalyst. Distillation or evaporation of the polar organic solvent from the extract solution may then be used for concentration and separation of the desired material. Another method is to add an aqueous alkali solution such as alkali hydroxide or alkali carbonate (or a mixture of aqueous alkali solution and water-soluble solvent), to the product treated with an acid catalyst, for extraction by stirring or shaking. After it is separated into an oil layer and a water layer or a layer of the aforementioned mixture (only the case of the water layer is considered hereinafter), a mineral or organic

acid is added to the water layer to neutralize it. An additional amount of acid is then introduced to make it an acidic system, and an organic solvent insoluble in water, such as ethyl acetate, diethyl ether or xylene, is used to extract the desired material. The solvent is finally distilled or evaporated away to complete the concentration and separation.

A crude distillate may be obtained by introducing steam at 100—200°C under normal or reduced pressure into a system processed with an acid catalyst. Concentration and separation of the desired material by removing free fatty acid and glycerides from the crude distillate may be performed by the aforementioned extraction methods. In addition molecular distillation of the processed material from the acid catalyst step may also be performed.

The desired material may also be obtained using several methods based on adsorption. Firstly, the desired material is adsorbed to the solid catalyst component and may thus be obtained by filtering or centrifugal separation followed by the aforementioned extraction methods for concentration and separation. Another method is to use an adsorbent, such as silica gel, silica/alumina, alumina or anion exchange resin activated charcoal, either in the presence or absence of an organic solvent to carry out an adsorption process (to be repeated several times, if necessary) on the product after the aforementioned treatment with an acid catalyst and then to concentrate and separate the desired material from this adsorbent by the aforementioned extraction method. A further method is to use an adsorbent at the same time as a solid catalyst is used for the acid catalyst treatment and to concentrate and separate the desired material from this adsorbent by the aforementioned extraction methods.

Of the extraction, distillation and/or adsorption methods described above, it is preferable to use one or more of the following methods for the concentration and separation of the phenol-type natural antioxidants: separation of triglyceride by cooling and precipitation in the presence of a polar organic solvent ((r) of Example 6 below), simultaneous or separate extraction using a non-polar organic solvent (such as hydrocarbon-type organic solvents) and an aqueous polar organic solvent (such as a mixture of water and lower alcohol and preferably alkaline) ((o) of Example 3, (p) of Example 4, (q) of Example 5 and (s) of Example 7 below), or molecular distillation ((s) of Example 7 below).

By qualitative analysis, the phenol-type natural antioxidant concentrated and separated by a method of the present invention is found to contain analogs of sesamin (A, B and C) and sesamolin (D) having the following chemical structures:

(A)

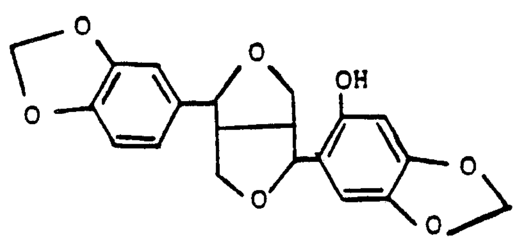

(B)

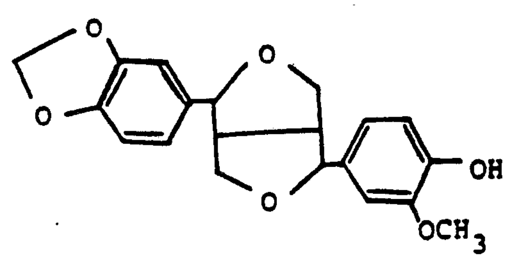

(C)

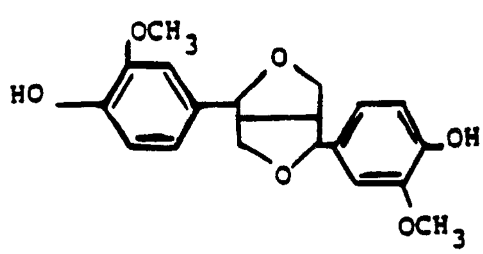

(D)

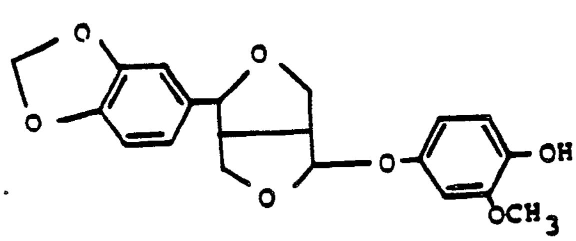

Of the above, A, B and D have been named earlier. The compound C is tetrahydro-1,4-bis[3-methoxy-4-hydroxyphenyl]-1H,3H-furo[3,4-C]furan (pinoresinol).

In a preferred embodiment, the present invention provides an antioxidant containing between 40 and 80% by weight of compound A, or between 41 and 78% of A when the antioxidant contains one or more of compounds A, B, C and D.

Further details of the present invention and its effects are given below by way of non-limiting examples.

Effects on Processed Sesame Seeds of Processing with Acid Catalysts

Acetone-extracted raw sesame seed oil (S—1) was prepared be pressing raw sesame seeds and distilling the acetone extract at a reduced pressure and at a temperature below 40°C to remove acetone. Separately, pressed raw sesame seed oil (S—2) was prepared by pressing similar raw sesame seeds to extract oil and filtering. Further, pressed raw sesame seed oil processed with acid catalyst (S—3) was prepared separately by adding one weight percent of activated clay to the pressed raw sesame seed oil (S—2) and filtering it after stirring the mixture for one hour at 70°C. Table 1 shows the results of analysis of S—1, S—2 and S—3.

TABLE 1

| | A | B | C | D | E | F |
|---|---|---|---|---|---|---|
| S—1 | ND | 3.7 | 4.1 | 1.5 | 48.8 | ND |
| S—2 | ND | 1.3 | 3.6 | 1.1 | 47.9 | T |
| S—3 | 81.9 | 8.1 | 9.0 | 11.2 | 25.5 | T |

In Table 1, the numbers are in units of %wt × $10^3$ A—D are the compounds described before. E and F represent r-tocopherol and sesamol, respectively. ND and T signify "not detected" and "traces only", respectively. The analyses were carried out by high-speed liquid chromatography (column: Dvelosil, ODS—10, 8 mm diameter × 250 mm) as follows. For A, B, C and D, the solvent was methanol/water = 6/4, the flow rate was 5 ml/min and the retention time was 11.8 min, 6.4 min, 2.7 min and 8.6 min respectively. For E, the solvent was methanol, the flow rate was 5 ml/min and the retention time was 8.5 min. For F, the solvent was methanol/water = 3.7, the flow rate was 4 ml/min, and the retention time was 10.4 min.

Example 1

A extractor was used to extract oil from raw sesame seeds from China and pressed raw sesame seed oil (a) was obtained by filtering. Next, coexisting free fatty acids were removed by washing with a caustic soda solution and also with water. Refined sesame seed oil (b) thus obtained (100 kg) was dehydrated under a reduced pressure at 70°C and 1 kg of acid clay was added as an acid catalyst. After 30 minutes of processing at 70°C, the mixture was filtered to obtain 98 kg of oil processed by acid catalyst (c) and 2 kg of acid clay filtered off residue (d).

Steam at 150—160°C was introduced into (c) for distillation under a reduced pressure. Steam distilled sesame seed oil (e) and 0.95 kg of steam distillation residue (f) were obtained. Separately, 100 g of (d) were placed in a 500 ml conical flask, 300 ml of ethyl acetate were added and the mixture was heated on a bath at 60°C for 30 minutes with an air condenser. After being cooled to room temperature, acid clay residue was filtered off and ethyl acetate was distilled off under a reduced pressure to obtain 39 g of ethyl acetate extract (g). By similar processes, 17.5 g of n-hexane extract (h), 22.0 g of acetone extract (i) and 5.1 g of ethanol extract (j) were also obtained from (d). Table 2 shows the results of analysis of (g), (h), (i) and (j) in units of %wt. Letters A—F are as explained for Table 1.

Example 2

100 g of (f) of Example 1 were placed in a 500 ml conical flask and 300 ml of ethyl acetate added. The mixture was heated on a bath at 60°C for 30 minutes with an air condenser. The mixture was left at 10°C overnight, filtered and solvent removed to obtain 80 g of ethyl acetate extract (k). By similar processes, 49 g of methanol extract (l) and 61 g of ethanol extract (m) were obtained. Table 2 also shows the results of analysis of (k), (l) and (m).

Example 3

100 g of (e) of Example 1 were dissolved in 200 ml of n-hexane, and 200 ml of methanol were added. The mixture was shaken and then left to separate a methanol layer to obtain 3.0 g of extract (n). Separately therefrom, 100 g of (e) of Example 1 were dissolved in 200 ml of n-hexane, 140 ml of ethanol and 60 ml of water were added and the mixture was shaken, and then left to separate the ethanol/water layer. Solvent

was removed from this ethanol/water layer to obtain 0.32 g of extract (o). Table 2 also shows the results of analysis of (n) and (o).

Example 4

100 g of (e) of Example 1 were dissolved in 200 ml of n-hexane after 140 ml of ethanol and 60 ml of 0.3 N aqueous caustic soda were added, the mixture was shaken, and then left to separate an ethanol/water layer. Hydrochloric acid was added to adjust the pH value of this ethanol/water layer to 2 and after 200 ml of ethyl ether were added, the mixture was shaken and the separated ethyl ether layer washed until the washing water became neutral. Anhydrous sodium sulfate was used for dehydration, and was filtered off. Solvent was removed to obtain 0.77 g of extract (p). Table 2 shows the results of analysis of (p).

Example 5

Raw sesame seeds similar to those used in Example 1 were roasted and then oil was extracted and filtered. Into 100 g of roasted sesame seed oil thus obtained was added 1 g of aluminum chloride and the mixture was heated and stirred for 30 minutes at 70°C. After it was cooled to room temperature, 200 ml of hexane, 100 ml of 0.5 N aqueous caustic soda and 100 ml of ethanol were added, and the mixture was stirred well to separate an ethanol/water layer. Hydrochloric acid was added to adjust the pH value of this ethanol/water layer to 2 and after 100 ml of xylene were added and the mixture shaken, the xylene layer obtained by leaving the mixture to stand was washed with water until the washing water became neutral. Anhydrous sodium sulfate was used for dehydration, and then filtered off. Solvent was removed to obtain 2.2 g of extract (q). Table 2 shows the results of analysis of (q).

Example 6

A paste-like material was obtained by crushing in a mill 1 kg of the raw sesame seeds as used in example 1. 3 l of methanol were added to the material and the mixture was stirred well. The extract solution obtained by filtering was left overnight at −20°C causing triglyceride to be deposited. After the solution was filtered 10 g of 35% hydrochloric acid were added. After 30 minutes in a reflux, it was cooled to room temperature, 2 l of xylene and 1 l of water were added and the mixture was shaken to separate a xylene layer. This xylene layer was washed with water until the washing water became neutral and solvent was removed to obtain 8.1 g of extract (r). Table 2 shows the results of analysis of (r).

Example 7

100 kg of the acid catalyst processed oil (c) obtained by the method described in Example 1 were subjected to molecular distillation at 210°C and 533 Pa (4mm Hg) to obtain 0.8 kg of distillate. This distillate was dissolved in 4 l of xylene. After 1 l of 0.5 N water solution of caustic soda and 3 l of isopropyl alcohol were added and the mixture was shaken well, it was left to stand and an isopropyl alcohol/water layer was obtained by separation. Hydrochloric acid was added to adjust its pH value to 2 and 2 l of xylene were used for extraction. After the xylene layer was washed with water until the washing water became neutral, solvent was removed to obtain 220 g of xylene extract. This xylene extract was dispersed in 1 l of hexane and after 30 minutes in a reflux, it was left to stand and the hexane layer filtered off to obtain 21 g of residue(s) insoluble in hexane. Table 2 also shows the results of analysis of (s).

TABLE 2

| | A | B | C | D | E | F |
|---|---|---|---|---|---|---|
| (g) | 0.77 | 0.07 | 0.07 | 0.12 | 0.06 | T |
| (h) | 0.06 | 0.01 | T | 0.02 | 0.05 | T |
| (i) | 0.68 | 0.07 | 0.09 | 0.12 | 0.05 | T |
| (j) | 1.13 | 0.15 | 0.27 | 0.34 | 0.05 | 0.01 |
| (k) | 0.82 | 0.04 | 0.05 | 0.11 | 0.52 | 0.02 |
| (l) | 1.02 | 0.05 | 0.07 | 0.18 | 0.50 | 0.03 |
| (m) | 0.91 | 0.05 | 0.07 | 0.18 | 0.61 | 0.02 |
| (n) | 1.85 | 0.19 | 0.20 | 0.31 | 0.04 | ND |
| (o) | 8.57 | 0.84 | 0.92 | 0.65 | T | 0.01 |
| (p) | 12.15 | 0.89 | 1.25 | 1.33 | ND | T |
| (q) | 10.10 | 0.72 | 1.29 | 1.00 | ND | 0.28 |
| (r) | 7.14 | 0.82 | 0.99 | 1.42 | T | T |
| (s) | 52.70 | 8.60 | 10.10 | 10.30 | T | 0.20 |

Tests of Antioxidative Characteristics

As examples of phenol-type natural antioxidants, 100 mg of (g), 40 mg of (o), 40 mg of (q) and 4 mg of (s) were separately placed in 100 ml conical flasks. Likewise, 4 mg of dl-α-tocopherol and 40 mg of commercially available natural antioxidant (SP—10 by Lion McCormick Company) were individually placed in flasks and 20 g of soya bean oil refined through a base alumina column were added to each of these flasks which were subsequently shaken well. They were kept in an oven at 98°C and the amount of peroxide (meg/kg) was measured over a period of time according to the usual procedure. The results are shown in Table 3.

TABLE 3

| | 0 hr | 3 hr | 5 hr | 7 hr | 10 hr | 15 hr |
|---|---|---|---|---|---|---|
| (g) | 3.7 | 11 | 17 | 26 | 55 | 156 |
| (o) | 3.7 | 10 | 17 | 23 | 37 | 80 |
| (g) | 3.7 | 10 | 16 | 23 | 34 | 57 |
| (s) | 3.7 | 12 | 18 | 26 | 42 | 69 |
| dl-α-tocopherol | 3.7 | 12 | 18 | 25 | 38 | 101 |
| commercial natural antioxidant | 3.7 | 11 | 28 | 54 | 112 | 200+ |
| no addition | 3.7 | 62 | 102 | 143 | 200+ | — |

The examples and experimental results (Tables 1—3) shown above make it clear that the present invention provides an industrially applicable method for manufacturing phenol-type natural antioxidants. The method includes acid catalyst processing and subsequent concentration-separation steps and results in phenol-type natural antioxidants containing a large amount of analogs of sesamin or sesamolin which exhibit excellent antioxidative characteristics. In particular the compound shown by A is produced in a large quantity.

**Claims**

1. A method of manufacturing phenol-type natural antioxidants comprising the steps of treating an oil or paste product derived from sesame seeds with an acid catalyst, and subsequently concentrating and separating said materials by physical means.

2. A method according to claim 1 wherein the antioxidant obtained contains one or more of the compounds shown by A, B and C:

(A)

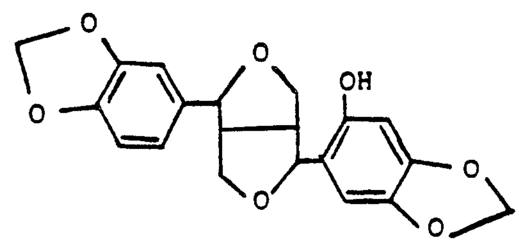

(B)

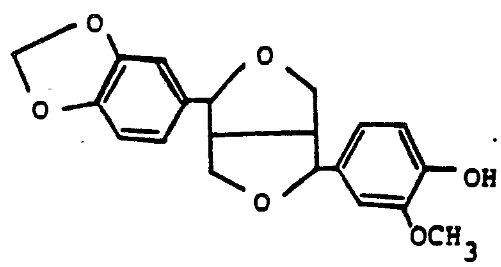

(C)

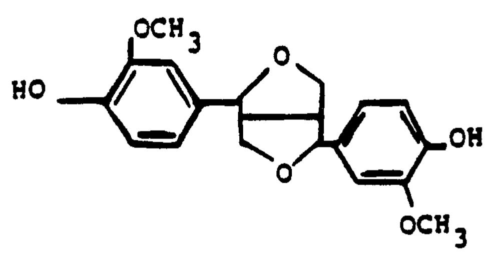

3. A method according to claim 1 wherein the antioxidant obtained contains a compound shown by the chemical formula D below:

(D)

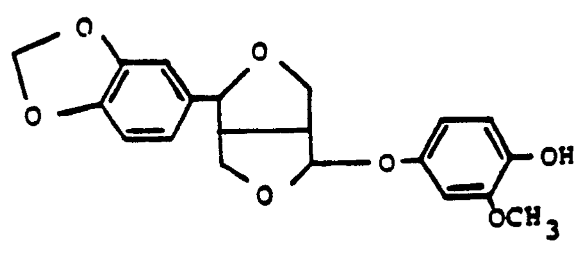

4. A method according to claim 2 wherein the antioxidant contains as its principal component a compound shown by the chemical formula A.

5. A method according to any of the preceding claims wherein the acid catalyst is a Bronsted acid, Lewis acid or solid catalyst having acid catalyst functions.

6. A method according to claim 5 wherein the Bronsted acid is selected from: hydrochloric acid, sulphuric acid, phosphoric acid, boric acid and p-toluene sulfonic acid.

7. A method according to claim 5 wherein the Lewis acid is selected from: aluminium chloride, titanium chloride, tin chloride and boron trifluoride.

8. A method according to claim 5 wherein the solid catalyst having acid catalyst functions is selected from acid clay, zeolite, silica-titanium oxide and cation exchange resin.

9. A method according to any of the preceding claims wherein the concentration and separation is achieved by one or more of the following means: separation of triglyceride by cooling and precipitation in the presence of a polar organic solvent, simultaneous or separate extraction using a non-polar organic solvent and an aqueous polar organic solvent, or molecular distillation.

10. An antioxidant containing between 40 and 80 percent by weight of a compound shown by A below:

(A)

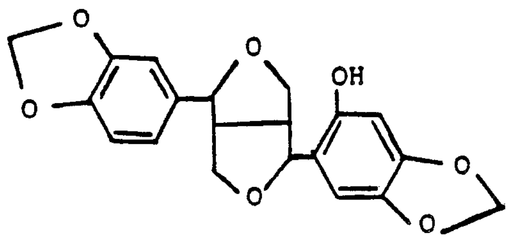

11. An antioxidant containing one or more of the compounds A, B, C and D as shown in claims 2 and 3, γ-tocopherol and sesamol characterised in that the percentage weight of A out of the total of A, B, C, D, γ-tocopherol and sesamol is between 41 and 78.

**Patentansprüche**

1. Verfahren zur Herstellung natürlicher Antioxidantien vom Phenol-Typ, umfassend die Stufen des Behandelns eines aus Sesamsamen gewonnenen Öls oder pastösen Produkts mit einem Säurekatalysator und des nachfolgenden Konzentrierens und Abtrennens der genannten Materialien mit tels physikalischer Methoden.

2. Verfahren nach Anspruch 1, bei dem das erhaltene Antioxidans eine oder mehrere der Verbindungen A, B und C enthält:

(A)

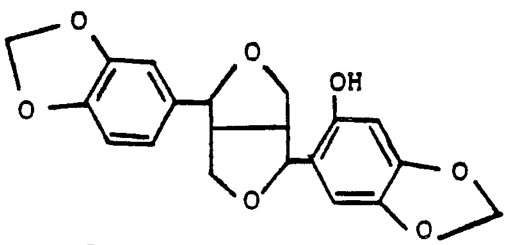

(B)

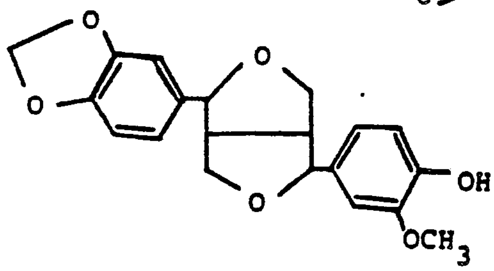

(C)

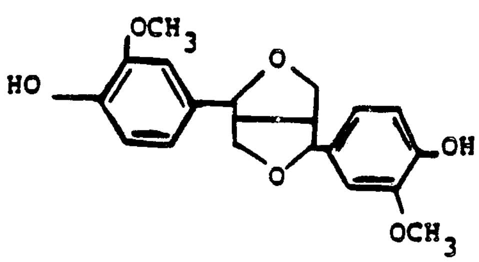

3. Verfahren nach Anspruch 1, bei dem das erhaltene Antioxidans eine Verbindung der nachfolgend wiedergegebenen chemischen Formel D enthält:

(D)

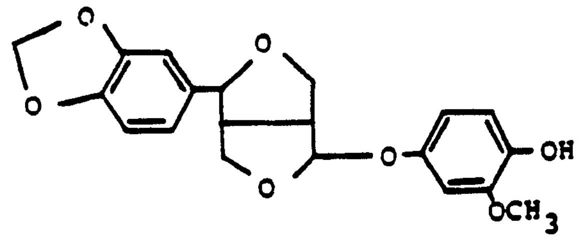

4. Verfahren nach Anspruch 2, bei dem das Antioxidans als Hauptkomponente eine Verbindung der chemischen Formel A enthält.

5. Verfahren nach einem der vorhergehenden Ansprüche, bei dem der Säurekatalysator eine Brönsted-Säure, eine Lewis-Säure oder ein fester Katalysator mit säurekatalytischer Wirkung ist.

6. Verfahren nach Anspruch 5, bei dem die Brönsted-Säure ausgewählt wird aus: Salzsäure, Schwefelsäure, Phosphorsäure, Borsäure und p-Toluolsulfonsäure.

7. Verfahren nach Anspruch 5, worin die Lewis-Säure ausgewählt wird aus: Aluminiumchlorid, titanchlorid, Zinnchlorid und Bortrifluorid.

8. Verfahren nach Anspruch 5, bei dem der feste Katalysator mit säurektalytischer Wirkung ausgewählt wird aus: Säureaktiviertem Ton, Zeolith, Siliziumdioxid-Titanoxid und Kationen austauschendem Harz.

9. Verfahren nach einem der vorhergehenden Ansprüche, bei dim das Konzentrieren und Abtrennen nach einer oder mehreren der folgenden Methoden durchgeführt wird: Abtrennen von Triglycerid durch Kühlen und Ausfällen in Gegenwart eines polaren organischen Lösungsmittels, gleichzeitige oder getrennte Extraktion unter Verwendung eines nichtpolaren organischen Lösungsmittels und eines wässerigen polaren organischen Lösungsmittels oder Molekulardestillation.

10. Antioxidans, enthaltend zwischen 40 und 80 Gewichtsprozent einer Verbindung der nachfolgend wiedergegebenen Formel A:

(A)

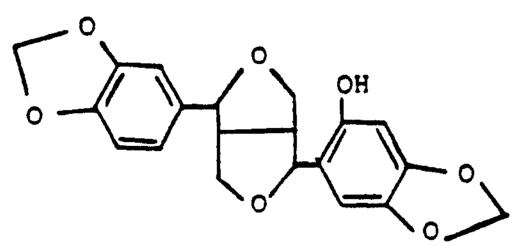

11. Antioxidans, enthaltend eine oder mehrere der Verbindungen A, B, C und D gemäß den Ansprüchen 2 und 3, γ-Tocopherol und Sesamol, dadurch gekennzeichnet, daß der Gewichtsprozentsatz von A, bezogen auf die Gesamtmenge an A, B, C, D, γ-Tocopherol und Sesamol, zwischen 41 und 78 beträgt.

**Revendications**

1. Procédé de préparation d'antioxydants phénoliques naturels, comprenant les étapes consistant à traiter une huile ou un produit pâteux dérivé de graines de sésame, avec un catalyseur acide, et à concentrer ainsi qu'à séparer ensuite, ces substances en mettant en oeuvre des moyens physiques.

2. Procédé selon la revendication 1, dans lequel l'antioxydant obtenu, contient un ou plusieurs des composés illustrés par les formules A, B et C:

(A)

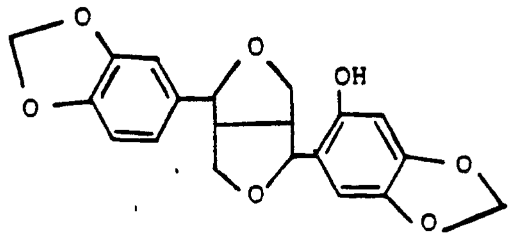

(B)

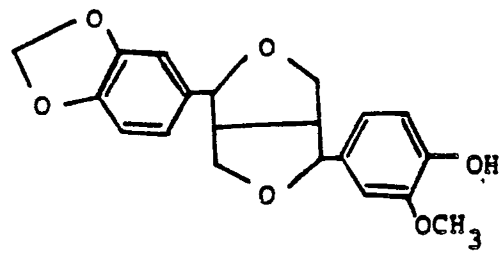

(C)

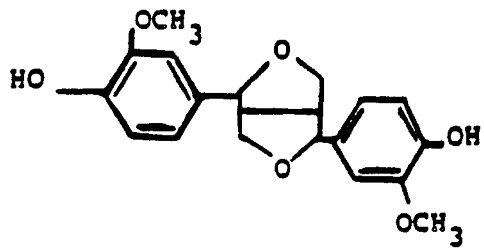

3. Procédé selon la revendication 1, dans lequel l'antioxydant obtenu contient un composé représenté par la formule chimique D ci-dessous:

(D)

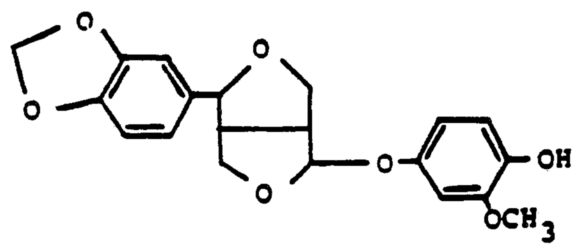

4. Procédé selon la revendication 2, dans lequel l'antioxydant, contient, comme composant principal, un composé représenté par la formule chimique A.

5. Procédé selon l'une quelconque des revendications précédentes, dana lequel le catalyseur acide, est un acide de Bronsted, un acide de Lewis ou un catalyseur solide ayant les activités d'un catalyseur acide.

6. Procédé selon la revendication 5, dans lequel l'acide de Bronsted est choisi parmi l'acide chlorhydrique, l'acide sulfurique, l'acide phosphorique, l'acide borique ainsi que l'acide p-toluène sulfonique.

7. Procédé selon la revendication 5, dans lequel l'acide de Lewis est choisi parmi le chlorure d'aluminium, le chlorure de titane, le chlorure d'étain et le trifluorure de bore.

8. Procédé selon la revendication 5, dans lequel le catalyseur solide ayant les activités d'un catalyseur acide, est choisi parmi une argile acide, une zéolite, de la silice associée à de l'oxyde de titane ainsi qu'une résine échangeuse de cations.

9. Procédé selon l'une quelconque des revendiations précédentes, dans lequel la concentration et la séparation, sont effectuées selon une ou plusieurs des techniques suivantes: séparation des triglycérides par refroidissement et précipitation en présence d'un solvant organique polaire, extraction simultanée ou séparée en utilisant un solvant organique apolaire et un solvant organique polaire aqueux, ou distillation moléculaire.

10. Antioxydant contenant entre 40 et 80 pour cent en poids d'un composé représenté par la formule A ci-dessous:

(B)

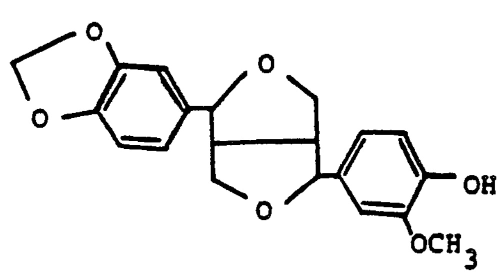

11. Antioxydant contenant un ou plusieurs des composés A, B, C, et D illustrés dans les revendications 2 et 3, le γ-tocophénol et le sêsanol, caractérisé en ce que le pourcentage en poids de A par rapport au total de A, B, C, D, γ-tocophénol et sêsamol est entre 41 et 78.